## Europäisches Patentamt
## European Patent Office
## Office européen des brevets

(19)

(11) Numéro de publication: **0 142 473**
**B1**

(12)
# FASCICULE DE BREVET EUROPÉEN

(45) Date de publication du fascicule du brevet:
**13.09.89**

(51) Int. Cl.⁴: **C 12 M 1/00**, C 12 P 5/02

(21) Numéro de dépôt: **84810541.7**

(22) Date de dépôt: **07.11.84**

(54) **Procédé de production de biogaz et de compost.**

(30) Priorité: **09.11.83 CH 6033/83**

(43) Date de publication de la demande:
**22.05.85 Bulletin 85/21**

(45) Mention de la délivrance du brevet:
**13.09.89 Bulletin 89/37**

(84) Etats contractants désignés:
**AT BE DE FR GB LU**

(56) Documents cité:
**EP-A-0 023 176**
**FR-A-2 266 739**
**FR-A-2 398 110**
**FR-A-2 498 044**

(73) Titulaire: **Cotton, Armand, 183 route de Mon Idée, CH- 1253 Crête- Vandoeuvres Genève (CH)**

(72) Inventeur: **Cotton, Armand, 183 route de Mon Idée, CH- 1253 Crête- Vandoeuvres Genève (CH)**

(74) Mandataire: **Munzinger, John Patrick, CH- 1254 Jussy l'Eglise Geneva (CH)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

EP 0 142 473 B1

## Description

La présente invention a pour premier objet un procédé de production de biogaz et de compost.

Pour produire du biogaz avec de la matière organique fermentable solide, tel que du fumier, il faut que cette matière subisse une fermentation dite méthanique (c'est-à-dire produisant du méthane, principal constituant du biogaz). Une telle fermentation ne peut se produire que dans un milieu anaérobie (sans air) et exige une certaine température qui varie selon que l'on veut avoir une fermentation en régime psychrophile (entre 10 et 22°C), mésophile (entre 22 et 45°C) ou thermophile (entre 45 et 75°C). Le milieu anaérobie est obtenu en noyant la matière organique dans une cuve avec un liquide. La température requise est obtenue, pour une fermentation en régime mésophile et une fermentation en régime thermophile, en chauffant la masse de matière et de liquide.

Comme on le sait, les matières organiques fermentables solides, tel le fumier, lorsqu'elles ne sont que simplement humides et qu'elles sont exposées à l'air, fermentent avec une augmentation rapide et relativement élevée de la température. Il s'agit d'une fermentation dite aérobie, faisant intervenir des micro-organismes (bactéries) différents de ceux responsables de la fermentation méthanique (ou anaérobie) énoncée plus haut. Contrairement à cette dernière, la fermentation aérobie est exothermique (génératrice de chaleur) et ne dégage pas de biogaz.

Ce phénomène de dégagement de chaleur a été mis à profit par Messieurs Gilbert Ducellier et Marcel Isman dans leur brevet français 893 767 du 28 mars 1942 pour la mise en route d'un processus de production de biogaz à température de fermentation mésophile afin d'une part de conditionner la matière pour qu'elle puisse subir une fermentation anaérobie, vu que la mise directe en fermentation anaérobie de la matière fraîche aboutit invariablement, après une courte période de production d'hydrogène, à une acidification du milieu provoquant un arrêt définitif de la fermentation, et d'autre part d'éviter un apport de chaleur extérieure.

Toutefois, lorsqu'il fait froid, la chaleur dégagée lois d'une fermentation aérobie est insuffisante, en tout cas au moment de la mise en route du processus de production de biogaz, pour porter la température du liquide à une valeur suffisamment élevée par échange de chaleur avec la matière solide échauffée pour permettre à la fermentation anaérobie de se faire dans des conditions acceptables.

Le procédé selon l'invention permet d'effectuer une fermentation anaérobie en régime mésophile à faible température ambiante dans de bonnes conditions. Ce procédé est défini dans les revendications 1 à 4.

Aux dessins schématiques annexés, donnés à titre d'exemple:

la figure 1 est une vue d'ensemble, en coupe longitudinale, d'une forme d'exécution de l'installation pour la mise en oeuvre du procédé selon l'invention;

la figure 2 est une vue en perspective d'un fermenteur utilisé dans l'installation de la figure 1 muni d'un fond modifié; et

la figure 3 est une vue en coupe horizontale du fermenteur représenté à la figure 2, muni d'une porte sans gonds.

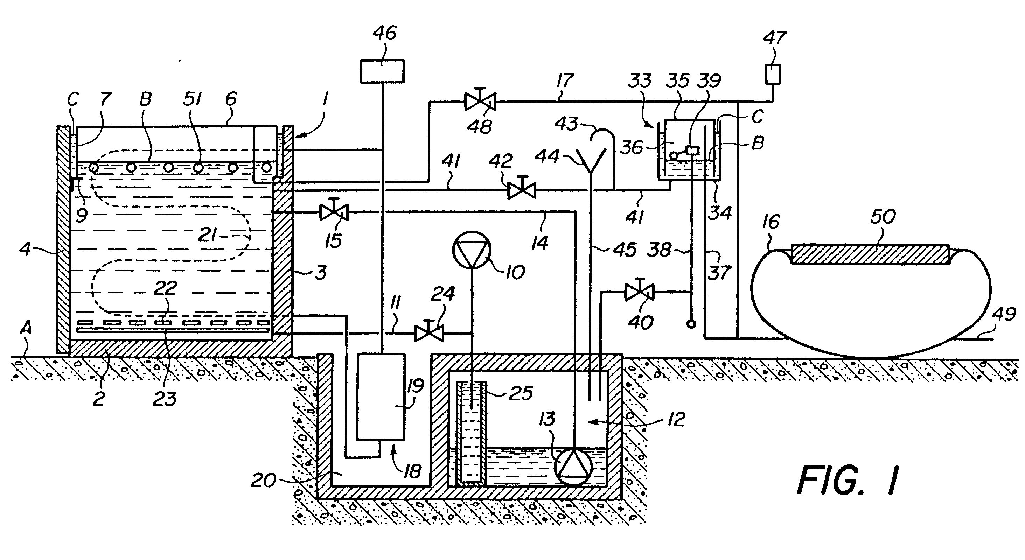

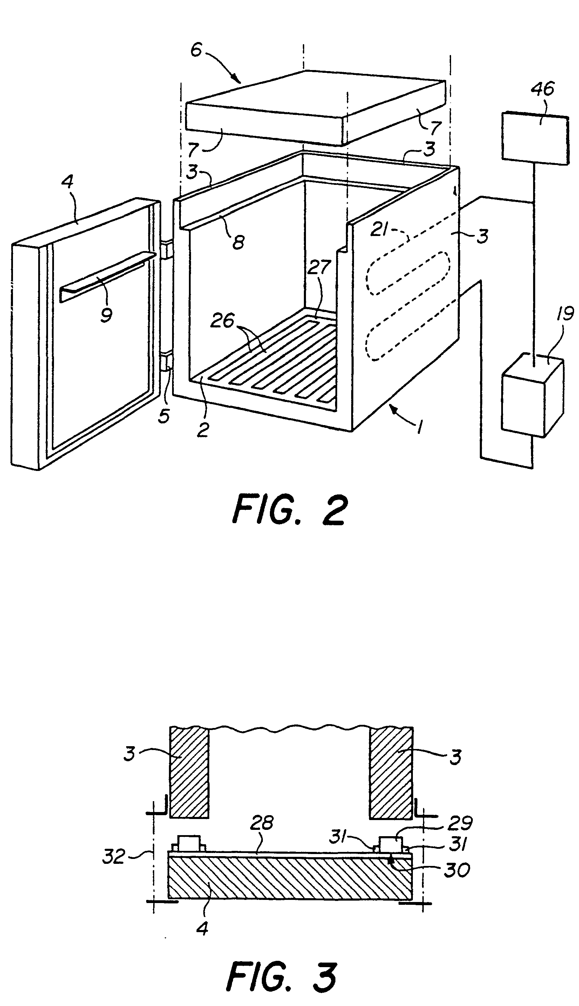

L'installation représentée à la figure 1 comprend un fermenteur (aussi appelé digesteur) reposant à même le sol A et constitué par une cuve parallélipipédique 1 en béton, en métal ou tout autre matériau étanche et résistant à la pression. La cuve utilisée ici a un volume d'environ 4,5 m³ avec un fond d'environ 3 m². Comme on le voit à la figure 2, la cuve comporte quatre éléments fixes, soit un fond 2 et trois parois verticales 3, ainsi qu'une porte légère 4, étanche et résistant à la pression, pouvant s'ouvrir, par rotation sur des gonds ou charnières 5 ou de toute autre manière. La cuve 1 est en outre munie d'un couvercle monobloc étanche 6, en tôle, en matière plastique ou autre matériau idoine, présentant des bords 7 tournés vers le bas et prenant appui sur des épaulements horizontaux 8 ménagés dans un même plan près du haut des trois parois 3 du côté intérieur de ceux-ci, et sur un rebord horizontal 9 fixé à l'intérieur de la porte au niveau des épaulements 8.

L'installation représentée convient particulièrement à une petite exploitation agricole pour la production de gaz à partir de fumier (notamment bovin, équin et porcin) et autres matières organiques fermentables solides provenant d'une telle exploitation (paille, fanes, herbe, feuilles, sciure, copeaux, etc.).

Pour la mise en service de cette installation on procède par exemple de la manière suivante:

1. On dégage le couvercle 6 et on ouvre la porte 4.

2. On remplit la cuve 1 de fumier pailleux frais, par exemple, à l'aide d'un tracteur équipé à l'avant de moyens de manutention, tels qu'une fourche, le tracteur accédant à l'intérieur de la cuve par la porte 4. Chaque fourchée est de préférence étalée, tassée, surtout près des bords, et, si la matière est plutôt sèche, mouillée. S'il s'agit de fumier bovin, aucun ensemencement de bactéries méthanogènes est nécessaire, ces bactéries étant naturellement présentes dans les fumiers de ruminants. Dans les autres cas, un ensemencement est nécessaire soit par du fumier bovin, des boues d'épuration ou des produits biologiques spécialement développés à cet effet.

3. On ferme et verrouille la porte 4 et on complète le chargement par le haut en tassant contre la porte et en s'assurant que les épaulements 8 et le rebord 9 sont dégagés.

4. On insuffle de l'air dans la cuve 1 par son fond 2 à l'aide d'un ventilateur 10 et une conduite 11 pendant environ 1 jour. Le substrat ayant été bien tassé contre les parois 3 et la porte 4, l'air est obligé de monter à travers la matière. Ceci provoque une fermentation (ou digestion) aérobie du substrat. Cette fermentation en fait monter la température, généralement a une valeur comprise entre 60 et 75°C, mais qui peut être encore plus élevée, notamment en son centre.

5. Au terme de cet échauffement, qui nécessite environ une journée, on arrête l'insufflation d'air et on noie le substrat chaud avec environ 3,5 m³ de purin et/ou d'eau à température ambiante (mettons 12°C) contenu dans une citerne 12, à l'aide d'une pompe de transfert 13 et une conduite 14 munie d'une vanne 15 à commande manuelle. La chaleur du substrat réchauffe alors le liquide d'environ 15°C. L'augmentation de température du liquide dépend de sa température de départ. Plus la température de départ est basse plus l'augmentation est grande.

6. Environ 2,5 m³ de liquide attiédi est alors évacué par le fond 2 et la conduite 11 pour être stocké dans la citerne 12. Le reste du liquide, soit environ 1 m³, est absorbé par le substrat.

7. Lorsque le substrat est suffisamment égoutté pour permettre à de l'air d'être insufflé (environ une demi-heure), on fait repartir la fermentation aérobie en remettant en marche le ventilateur 10 pour ramener la température du substrat à 60°C ou plus. Cette insufflation se poursuit pendant environ deux jours.

8. On noie de nouveau le substrat avec le liquide attiédi stocké dans la citerne 12. Comme la différence de température entre le substrat et le liquide est cette fois plus faible, la température du liquide augmente moins, soit d'environ 10 degrés.

9. Si le liquide à la fin de cette dernière opération de chauffage a atteint, comme cela serait le cas avec l'exemple donné, une température voisine de 35°C, qui est la température optimale pour une fermentation méthanique mésophile, on remet en place le couvercle 6 en le bloquant à l'aide de fixations non représentées, on laisse se dérouler la fermentation méthanique mésophile, laquelle pourra durer de quatre à huit semaines selon le but recherché, et on recueille le gaz dégagé dans un réservoir de stockage (parfois appelé gazomètre) 16 de volume variable par l'intermédiaire d'une conduite 17. Un but peut être une production maximale de biogaz; dans ce cas on arrête le processus de fermentation au bout de quatre semaines déjà pour mettre en route une nouvelle cuvée. Un autre but peut être plutôt axé sur la production de compost, auquel cas on fait durer la fermentation presque jusqu'à son terme, soit environ huit semaines. Des durées de fermentation intermédiaires sont bien entendu aussi envisageables.

10. Mais si le liquide à la fin de l'opération (8), après mettons environ un quart d'heure, n'a pas encore atteint une température voisine de 35°C (en hiver notamment), on répète les opérations (6), (7) et (8) le nombre de fois qu'il faut pour arriver à la température requise, avant de procéder à l'opération (9). Pour une troisième opération de fermentation aérobie, l'insufflation durera environ trois jours. Le gain de température du liquide ne sera cette fois que de 4 à 6°C. Pour une quatrième opération de fermentation (rarement nécessaire) le temps d'insufflation sera encore plus long et le gain de température du liquide sera encore plus faible.

Les temps indiqués ci-dessus sont tous approximatifs, valables seulement avec l'installation expérimentale décrite ici. Ces temps peuvent donc varier d'une installation à l'autre, et sont fonction du genre de substrat traité et de son humidité. Il en va de même avec les gains de température obtenus à la suite de chaque fermentation aérobie; ceux-ci dépendent aussi des conditions atmosphériques.

Cette suite d'opérations de fermentation aérobies (qu'on peut appeler de préfermentation) ne sont nécessaires que lors de la mise en service de l'installation pour amener le liquide requis pour la fermentation méthanique à la température voulue. Comme on peut le constater, ces opérations de préfermentation n'exigent aucun apport de chaleur et se font dans la même cuve que la fermentation méthanique.

Comme cela ressort de l'opération (6) il se produit une absorption importante de liquide lors de la première immersion du substrat. Lors de la deuxième immersion, il absorbera une quantité moindre de liquide et lors de la troisième immersion il absorbera une quantité de liquide moindre encore. De ce fait, la quantité de liquide devant être introduite dans la cuve 1 pour les immersions subséquentes diminue d'autant. Ainsi, il y a de moins en moins de liquide à chauffer.

On a trouvé qu'il est préférable d'obtenir une température de quelques degrés (par exemple 3°C) supérieure à celle de la température optimale de 35°C, afin de disposer d'une certaine marge de sécurité contre les déperditions de chaleur inhérentes à la mise en service.

A la fin de la fermentation méthanique, on enlève le couvercle 6, on soutire le liquide chaud pour le stocker dans la citerne 12, on ouvre la porte 4, on vidange la cuve 1 à l'aide d'un tracteur muni d'une fourche à fumier frontale, on répète les opérations (2) à (4), on arrête l'insufflation, on réintroduit dans la cuve 1 le liquide chaud, gorgé de ferments, stocké dans la citerne 12, après avoir ajouté au préalable du liquide d'appoint à température ambiante pour obtenir le volume nécessaire, on remet en place le couvercle 6, on laisse se dérouler une nouvelle fermentation méthanique mésophile, et on recueille de nouveau le gaz dégagé, cycle d'opérations qui peut être répété indéfiniment pendant la durée de vie de l'installation.

Pour réduire au minimum les déperditions de chaleur, il importe d'isoler autant que possible la cuve 1 et la citerne 12, par exemple avec des panneaux de polyuréthane et/ou de la laine de verre. Cependant, quelle que soit l'efficacité de l'isolation, il se produira néanmoins des pertes de chaleur. Pour compenser celles de la cuve 1 et ainsi maintenir la température optimale de 35°C à l'intérieur de celle-ci pendant la fermentation anaérobie mésophile (laquelle n'engendre que très peu de chaleur), l'installation est en outre équipée d'un thermosiphon 18 comportant un chauffe-eau 19 disposé au dessous du niveau du sol A (et donc de la cuve 1) dans une fosse 20, un ou plusieurs serpentins 21 conduisant le fluide caloporteur, qui sont noyés dans une ou plusieurs des parois 3 de la cuve 1, et un vase d'expansion 46. Les serpentins 21, en étant noyés dans les parois 3, permettent à un tracteur de manoeuvrer avec sa fourche dans la cuve 1 sans danger pour le dispositif de chauffage. La chaudière, qui consomme du biogaz pour maintenir la température optimale de production, n'utilise qu'une petite partie du gaz produit (environ 10 % en moyenne normale). Ce chauffage par thermosiphon économise la mise en place d'un circulateur et sa consommation d'énergie de fonctionnement.

Par ailleurs, lorsque les lieux s'y prêtent, il est possible de monter en série avec le chauffe-eau 19 un ou plusieurs capteurs solaires et d'améliorer ainsi le rendement énergétique de l'installation.

Lorsque le biogaz est utilisé comme combustible pour un moteur entraînant, par exemple, une génératrice d'électricité, on peut faire circuler l'eau de refroidissement de ce moteur dans les serpentins 21.

Comme on l'a vu, l'air insufflé dans la cuve 1 et le liquide qui en est recueilli s'écoulent à travers le fond 2 de la cuve. Ce fond peut être constitué, comme représenté à la figure 1, par des éléments amovibles 22 de caillebotis, en métal, en béton ou en bois, portés à une certaine distance au-dessus du radier de la cuve par des cornières 23 fixées aux parois 3, pour former un vide permettant, d'une part, au liquide d'être évacué par la conduite 11 dans la citerne 12 et, d'autre part, à l'air d'être sous tout le tas de substrat dans la cuve 1.

Comme on le voit à la figure 1, la conduite 11 comporte une partie sensiblement horizontale munie d'une vanne 24 à commande manuelle et une partie sensiblement verticale. Le haut de la partie verticale est connecté au ventilateur 10 tandis que le bas aboutit dans un récipient 25 constamment rempli de liquide, disposé dans la citerne 12. Cette partie verticale plonge dans le liquide du récipient 25 suffisamment profondément pour empêcher à de l'air provenant du ventilateur 10 de s'écouler par le bas de la partie verticale au lieu de s'écouler dans la cuve 1. D'autre part, le ventilateur 10 doit être placé à un niveau suffisamment élevé pour ne pas être atteint par le liquide s'écoulant hors de la cuve 1 dans la citerne 12 (par l'intermédiaire du récipient 25).

Lors du remplissage de la cuve 1 à l'aide de la pompe 13, et pendant toute la durée de fermentation méthanique, la conduite 11 est maintenue fermée à l'aide de la vanne 24.

Au début des préfermentations l'air et le liquide qui s'égoutte du fermenteur s'écoulent dans la partie horizontale de la conduite 11 en sens contraires.

Les éléments de caillebotis 22 étant coûteux, étant généralement peu durables et devant de surplus être démontés pour nettoyer le vide sous le fond 2 avant chaque rechargement, il est préférable de leur substituer un caillebotis fixe comme celui représenté à la figure 2.

Ce caillebotis fixe est réalisé beaucoup plus économiquement, sur place, au moment de la construction de la cuve. Il comporte un ensemble de rainures parallèles 26 d'environ 3 cm de large et de 6 à 8 cm de profondeur (pour obtenir une pente), reliées par une rainure collectrice transversale 27 de même largeur mais plus profonde, à l'arrière de la cuve et communiquant avec la conduite 11. Les rainures 26 sont espacées d'environ 7 cm les unes des autres.

Pour réaliser le caillebotis, on fabrique au préalable en matière facilement destructible ou extractible après coulée du béton (par exemple du polystyrène expansé) de moule (ou négatif) dont les pleins correspondent aux rainures 26 et 27 pour l'écoulement de l'air et du liquide.

Ce moule est ensuite posé sur une couche de base, grossièrement dressée, en béton armé frais formant le radier, et les vides sont alors soigneusement remplis avec du mortier.

Après la prise du béton et du mortier, la matière constituant le moule est enlevée pour laisser les rainures 26 et 27 dans un fond 2 carrossable.

Ce caillebotis faisant corps avec le radier, est très solide et durable. De plus, les rainures parallèles peuvent, sans nécessiter aucun démontage, être aisément nettoyées avec un racle de forme simple.

La porte 4 est réalisée en bois ou tout autre matériau léger et rigide. Son étanchéité est assurée à l'aide d'une plaqué rigide 28 de matière plastique, par exemple une plaque de PVC ayant une épaisseur d'environ 5 mm, recouvrant la face interne de la porte à laquelle elle est fixée par tous moyens appropriés tels que colle ou vis. Le rebord 9, constitué ici par une cornière, est soudé à la plaque 28.

L'étanchéité entre la porte 4 et le reste de la cuve 1 est assurée par un joint constitué par une bande continue 29 de mousse pincée dans un logement 30. La mousse est de préférence relativement rigide. Elle doit être à cellules occluses et doit pouvoir résister à l'attaque du purin. En l'occurrence, cette mousse est une mousse de caoutchouc naturel. Le logement 30 est soudé à la plaque 28 en regard de la tranche du radier de la cuve et de la tranche des deux parois 3 avoisinantes (figure 2) de façon que la mousse soit appliquée avec un certain écrasement contre ces tranches lors de la fermeture de la porte. Les tranches sont bien sûr bien planes. Le logement 30 peut par exemple être formé par un ou des éléments profilés rigides en matière plastique qui sont soudés de façon étanche contre la plaque 28 et qui présentent une rainure dans laquelle la bande de mousse 29 est pincée, ou, comme représenté à la figure 3, être constitué par des paires de nervures espacées 31 qui sont formées et disposées de façon à ménager une rainure venant pincer la bande 29 et qui sont soudées elles aussi contre la plaque. Ce pincement de la bande 29 permet à celle-ci d'être facilement remplacée.

La porte 4 est maintenue plaquée contre lesdites tranches par des fixations 32 (figure 2) disposées de façon assez rapprochée les unes des autres, ou de toute autre manière convenable.

L'étanchéité entre le couvercle 6, d'une part, et la cuve 1 et la porte 4, d'autre part, est assurée par un joint hydraulique formé par le liquide utilisé pour noyer le substrat dans la cuve 1, en relation avec les bords 7 du couvercle 6 lorsque celui-ci repose sur les épaulements 8 et le rebord 9, dans la mesure où le liquide est à un niveau supérieur à celui des épaulements 8 et du rebord 9 de part et d'autre des bords 7, et le demeure pendant toute la durée de la fermentation méthanique.

Le gaz qui se dégage du substrat s'accumule sous le couvercle 6 et crée une surpression. Cette surpression se manifeste par une baisse du niveau B du liquide à l'intérieur du couvercle 6 et une hausse du niveau C du liquide à l'extérieur du couvercle 6. Le gaz occasionnant ce surplus de pression est évacué vers le réservoir 16 par la conduite 17.

Le fonctionnement du joint hydraulique nécessite, d'une part, que le niveau B du liquide à l'intérieur du couvercle 6 ne descende pas au-dessous d'un certain niveau sinon le gaz s'échappe à l'extérieur en passant sous les bords 7 du couvercle 6, et, d'autre part, que le niveau C du liquide à l'extérieur du couvercle ne dépasse pas aussi un certain niveau sinon le fermenteur déborde.

Or, en pratique le niveau B du liquide dans le fermenteur varie en fonction des variations de volume du substrat en cours de fermentation (dues notamment à la digestion) et aussi des variations de la pression du gaz dans le réseau.

Pour maintenir, malgré ces variations, le niveau C du liquide à l'extérieur du couvercle 6 à un niveau maximal donné et le niveau B du liquide à l'intérieur du couvercle 6 à un niveau minimal donné, il est prévu un régulateur de niveau 33. Ce régulateur, disposé à l'extérieur du fermenteur, à la hauteur du couvercle 6, comprend un bac 34 et un couvercle 35 présentant un bord 36 venant reposer à l'intérieur du bac 34 sur le fond de celui-ci, fond qui se trouve au même niveau que les épaulements 8 et le rebord 9 de la cuve 1. Le bord 36 a la même hauteur que les bords 7 du couvercle 6 et entre les parois du bac 34 et le bord 36 est ménagé un espace analogue à celui ménagé entre le couvercle 6 et la cuve 1, ceci pour permettre la reconstitution à plus petite échelle du joint hydraulique du fermenteur avec ses différents niveaux B et C de liquide dus à la différence des pressions régnant à l'extérieur et à l'intérieur du couvercle 6.

Un tuyau 37, raccordé au réservoir de stockage 16 de l'installation, aboutit sous le couvercle 35 pour créer à l'intérieur de celui-ci une pression égale à celle régnant sous le couvercle 6 du fermenteur, une égalisation de pression étant déjà établie entre le réservoir 16 et le fermenteur par l'intermédiaire de la conduite 17. Un deuxième tuyau, 38, alimente en eau du réseau une vanne à flotteur 39 réglant le niveau minimal du liquide à l'intérieur du couvercle 35 et, au travers d'une vanne 40 à commande manuelle, la citerne 12.

Un troisième tuyau, 41, alimente en eau de complément la cuve de fermentation 1 à partir du bac 34. Ce tuyau 41 est muni d'une vanne 42 à commande manuelle qu'on ferme avant chaque déchargement de la cuve 1 et qu'on rouvre au début de chaque phase suivante de fermentation méthanique. Sur le tuyau 41 est en outre monté, entre le bac 34 et la vanne 42, un trop-plein 43 qui débouche à l'air libre et qui fixe le niveau maximal du liquide à l'extérieur du couvercle 35 et à l'extérieur du couvercle 6 grâce au tuyau 41, empêchant le bac 34 et la cuve 1 de déborder. Tout liquide issu du trop-plein 43 est recueilli dans un entonnoir 44 pour être amené dans la citerne 12 par l'intermédiaire d'un tuyau 45. Le couvercle 35 est maintenu en place par une fixation non représentée.

Le niveau maximal du liquide peut être atteint, d'une part, lorsqu'il se produit une dilatation de la masse contenue dans le fermenteur et, d'autre part, lorsqu'il se produit une augmentation de la pression du gaz dans le réseau.

Une dilatation de la masse normalement provoque une hausse égale à la fois des niveaux B et C du liquide à l'intérieur et à l'extérieur des couvercles 6 et 35 de la cuve 1 et du bac 34. Bien que la hausse du niveau C du liquide à l'extérieur des couvercles 6 et 35 est limitée grâce au trop-plein 43, le niveau B du liquide à l'intérieur des couvercles 6 et 35 peut cependant continuer à monter. La hauteur des bords 7 et 36 des couvercles 6 et 35 doit donc être dimensionnée pour tenir compte de la dilatation de masse maximale pour toujours laisser des espaces libres pour le gaz, en faisant en sorte que les extrémités supérieures de la conduite 17 et du tuyau 37 aboutissent dans ces espaces au-dessus du niveau maximal interne.

Une augmentation de la pression, qui se produit de toute manière au fur et à mesure du remplissage du réservoir 16 et qui peut en plus se produire lorsque le réservoir 16, ici fait en tissu caoutchouté, subit un échauffement dû au soleil, provoque une baisse du niveau B à l'intérieur des couvercles 6 et 35. Compte tenu du rapport des surfaces du liquide à l'intérieur et à l'extérieur des couvercles 6 et 35, une faible baisse du niveau B à l'intérieur des couvercles normalement provoque une forte hausse du niveau C à l'extérieur des couvercles. Ceci peut donner lieu à la fois à un déversement prolongé de liquide par le trop-plein 43 pour empêcher le niveau extérieur C du liquide de dépasser le niveau maximal et à une introduction prolongée de liquide dans le bac 34 par la vanne à flotteur 39 pour empêcher le niveau intérieur B du liquide de passer au-dessous du niveau minimal. Cet état de choses peut être évité en implantant dans le réseau de gaz une soupape de surpression 47, par exemple dans la conduite 17.

La conduite 17 est aussi munie, entre la soupape 47 et la cuve 1, d'une vanne 48 à commande manuelle, qu'on ferme avant d'enlever le couvercle 6 à la fin d'une fermentation méthanique pour éviter que le gaz stocké dans le réservoir 16 ne puisse s'échapper. Cette vanne 48 est de nouveau ouverte lorsque le couvercle 6 est remis en place pour la mise en route d'une nouvelle fermentation méthanique.

Le gaz stocké dans le réservoir 16 peut être acheminé par une conduite 49 vers différents points de consommation dont le brûleur de la chaudière 19. Pour cela il faut que le gaz ait une pression minimale, par exemple 8 millibars. A cette fin un lest 50 de poids approprié est posé sur le réservoir flexible 16. Ce lest assure cette pression minimale dès le début du stockage. Lorsque le réservoir est plein, la pression du gaz pourra, par exemple, atteindre environ 20 millibars.

Pendant la fermentation méthanique, le substrat que contient la cuve 1 tend à flotter. Pour empêcher celui-ci de monter à la surface du liquide, le couvercle 6 est équipé, au niveau de l'extrémité inférieure de ses bords 7, d'une série de barres ou tubes 51 formant une grille. En rendant cette grille solidaire (par soudure notamment) du couvercle 6, on évite toute manutention séparée de ces barres ou tubes de maintien en immersion du substrat. L'isolation thermique du couvercle 6 est également incorporée à ce dernier ce qui évite aussi une autre manipulation séparée.

Diverses modifications peuvent être apportées au procédé et à l'installation décrits ci-dessus en regard des dessins. Par exemple, on peut utiliser comme substrat, des ordures ménagères, triées pour en éliminer les matières non fermentables et non solides, et, comme liquide, des boues d'épuration.

Au lieu d'un seul fermenteur, l'installation peut comporter une batterie de plusieurs fermenteurs mis en parallèle sur le réservoir 16 pour assurer une production continue de gaz, le nombre de fermenteurs étant fonction de la quantité de matière organique fermentable disponible. Dans une telle installation un seul régulateur de niveaux suffit, pour autant que les niveaux de liquide sus-mentionnés soient les mêmes pour tous les fermenteurs.

Quant au choix du nombre de fermenteurs dans une telle installation, il est utile de prévoir une cuve en plus qui ne produit pas de biogaz (par exemple quatre fermenteurs lorsqu'on veut en avoir trois en production) ceci dans le but d'avoir à disposition une cuve pour y déposer journalièrement tout fumier frais. Ainsi, on évite la construction d'un tas de fumier extérieur devant par la suite être transféré dans une cuve une fois vidée de son contenu à la fin d'un cycle.

Dans ces conditions, la cuve non productrice peut aussi servir de contenant pour le liquide attiédi issu d'un fermenteur en train d'être mis en service. Ainsi, le liquide attiédi ne ferait que transiter brièvement dans la citerne 12 de sorte que le volume de celle-ci peut être sensiblement réduit, par exemple à 1 m³, quel que soit le volume des cuves 1 en batterie.

La conduite 17 et le tuyau 37, au lieu de traverser les parois de la cuve 1 et du bac 34, peuvent traverser le haut des couvercles 6 et 35. Dans ce cas, la conduite 17 et le tuyau 37 sont flexibles au moins au voisinage des couvercles pour permettre la manoeuvre de ceux-ci.

En pratique, les fermenteurs ont des volumes nettement plus grands qu'indiqués, pouvant atteindre plusieurs dizaines de mètres cubes.

**Revendications**

1. Procédé de production de biogaz et de compost, suivant lequel (1) on laisse fermenter en milieu aérobie de la matière organique fermentable solide à température sensiblement ambiante et ayant un degré d'humidité idoine pour l'amener à une température supérieure à celle exigée pour une fermentation méthanique mésophile en milieu anaérobie, et (2) on noie la matière organique en fermentation avec du liquide à température sensiblement ambiante pour augmenter la température du liquide par échange de chaleur avec la matière organique en fermentation, caractérisé en ce que (3) on évacue le liquide pour le stocker en un milieu isolé thermiquement en ramenant la matière à un degré d'humidité idoine, (4) on amène de nouveau la matière à ladite température supérieure par fermentation aérobie, (5) on noie de nouveau la matière avec le liquide stocké pour augmenter encore davantage la température de celui-ci par échange de chaleur avec la matière en fermentation, et (6) si le liquide noyant la matière n'a pas encore atteint la température exigée pour assurer une fermentation méthanique mésophile de la matière en milieu anaérobie on répète les opérations (3), (4) et (5) jusqu'à obtention de ladite température exigée, après quoi (7) on laisse en place le liquide une fois ladite température exigée atteinte pour permettre à la fermentation méthanique mésophile anaérobie de se développer, et (8) on recueille le gaz dégagé lors de cette fermentation anaérobie.

2. Procédé selon la revendication 1, suivant lequel ladite température exigée pour assurer une fermentation méthanique mésophile est comprise entre 34° et 40°C.

3. Procédé selon la revendication 1 ou 2, suivant lequel on aère la matière organique fermentable lors des étapes (1) et (4), notamment par insufflation.

4. Procédé selon l'une quelconque des revendications 1 à 3, suivant lequel la matière organique fermentable est en tas.

## Patentansprüche

1. Verfahren zur Herstellung von Biogas und Kompost, nach welchem man (1) feste gärungsfähige organische Materie im wesentlichen unter Umgebungstemperatur und mit einem geeigneten Feuchtigkeitsgehalt in aerobischer Umgebung gären lässt, um sie auf eine höhere als die für eine mesophile methanische Gärung in anaerobischer Umgebung erforderliche Temperatur zu bringen, und wobei man (2) die gärende organische Materie mit Flüssigkeit von hauptsächlich Umgebungstemperatur überschwemmt, um die Flüssigkeitstemperatur durch Wärmeaustausch mit der gärenden organischen Materie zu erhöhen, dadurch gekennzeichnet, dass man (3) die Flüssigkeit entfernt und sie in einer thermisch isolierten Umgebung lagert, um die Materie auf einen zweckmässigen Feuchtigkeitsgehalt zurückzubringen, wonach man (4) besagte Materie bei aerobischer Gärung wiederum auf besagte höhere Temperatur bringt, man (5) sie erneut mit der gelagerten Flüssigkeit überschwemmt, um deren Temperatur durch Wärmeaustausch mit der gärenden Materie weiter zu erhöhen und man (6) die Vorgänge (3) (4) und (5) wiederholt, falls die die Materie überschwemmende Flüssigkeit die für eine mesophile methanische Gärung in anaerobischer Umgebung erforderlichen Temperatur noch nicht erreicht hat, bis besagte erforderliche Temperatur erreicht ist, worauf man (7) die Flüssigkeit vor Ort belässt, sobald diese erforderliche Temperatur, die die mesophile methanische Gärung in anaerobischer Umgebung erzeugen kann, erreicht ist und man (8) das bei dieser anaerobischen Gärung erzeugte Gas einsammelt.

2. Verfahren nach Anspruch 1, wobei die obige erforderliche Temperatur für eine mesophile methanische Gärung zwischen 34° und 40°C liegt.

3. Verfahren nach Anspruch 1 oder 2, wobei man die gärungsfähige organische Materie bei den Vorgängen (1) und (4) lüftet, besonders durch Einblasen von Luft.

4. Verfahren nach irgend einem der Ansprüche 1 bis 3, wobei die organische Materie aufgehäuft wird.

## Claims

1. A method of producing biogas and compost, which comprises (1) fermenting in an aerobic environment solid fermentable organic matter at substantially ambient temperature and of appropriate moisture content to raise its temperature to a temperature greater than that required for mesophile methanogenic fermentation in an anaerobic environment, and (2) flooding the fermenting organic matter with liquid at substantially ambient temperature to increase the temperature of the liquid by heat exchange with the fermenting organic matter, characterized by (3) removing the liquid to store it in a heat insulated environment to reduce the moisture content of the matter to a suitable degree, (4) raising the temperature of said matter to said greater temperature by aerobic fermentation, (5) again flooding the matter with the stored liquid to raise the temperature of the latter still further by heat exchange with the fermenting matter, and (6) repeating steps (3), (4) and (5) if the liquid flooding the matter has not yet reached the temperature required for mesophile methanogenic fermentation of the matter in an anaerobic environment until said required temperature reached, thereupon (7) leaving the liquid in situ once said required temperature has been reached to enable anaerobic mesophile methanogenic fermentation to take place, and (8) collecting the gas given off during such anaerobic fermentation.

2. A method as in claim 1, wherein the temperature required for mesophile methanogenic fermentation lies between 34° and 40° C.

3. A method as in claim 1 or 2, which comprises ventilating the fermentable organic matter during steps (1) and (4) in particular by blowing in air.

4. A method as in any one of claims 1 to 3, wherein the fermentable organic material is heaped.

FIG. 1

FIG. 2

FIG. 3